(19) Europäisches Patentamt
European Patent Office
Office européen des brevets

(11) Veröffentlichungsnummer: **0 129 254**
**A2**

(12) # EUROPÄISCHE PATENTANMELDUNG

(21) Anmeldenummer: **84107071.7**

(22) Anmeldetag: **20.06.84**

(51) Int. Cl.³: **C 07 D 233/64**
C 07 K 5/08, A 61 K 37/02

(30) Priorität: **20.06.83 DE 3322117**

(43) Veröffentlichungstag der Anmeldung:
**27.12.84 Patentblatt 84/52**

(84) Benannte Vertragsstaaten:
**AT BE CH DE FR GB IT LI LU NL SE**

(71) Anmelder: **Diamalt Aktiengesellschaft**
**Friedrichstrasse 18**
**D-8000 München(DE)**

(72) Erfinder: **Voelter, Wolfgang, Prof. Dr.**
**Panoramastrasse 71**
**D-7400 Tübingen-Hageloch(DE)**

(54) **Verfahren zur Herstellung von N-tau-substituierten Histidinderivaten, N-tau-substituierte Histidinderivate und ihre Verwendung.**

(57) Es wird ein Verfahren zur Herstellung von $N^{\tau}$-substituierten Histidinderivaten der allgemeinen Formel

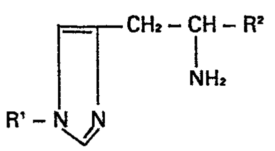

(1)

beschrieben. Gemäß dem Verfahren wird ein Histidinderivat der allgemeinen Formel

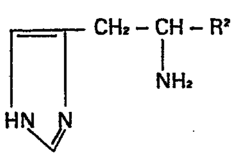

(2)

mit Phosgen umgesetzt, und das erhaltene Produkt wird dann alkyliert und anschließend mit Säure hydrolysiert.

Es werden ebenfalls die bei dem Verfahren erhaltenen Produkte und ihre Verwendung beschrieben.

EP 0 129 254 A2

Croydon Printing Company Ltd.

## BESCHREIBUNG

Die Erfindung betrifft ein Verfahren zur Herstellung von $N^{\tau}$-substituierten Histidinderivaten, $N^{\tau}$-substituierte Histidinderivate und ihre Verwendung zur Herstellung von Peptidderivaten, insbesondere hochaktiven Hormonderivaten, und Enzymderivaten.

Der Nachweis der beiden isomeren Aminosäuren 1-Methylhistidin der folgenden Formel 6 und 3-Methylhistidin der folgenden Formel 7, nach IUPAC-Nomenklatur $N^{\pi}$-(pros)- und $N^{\tau}$-(tele)-Methylhistidin gelang schon relativ früh:

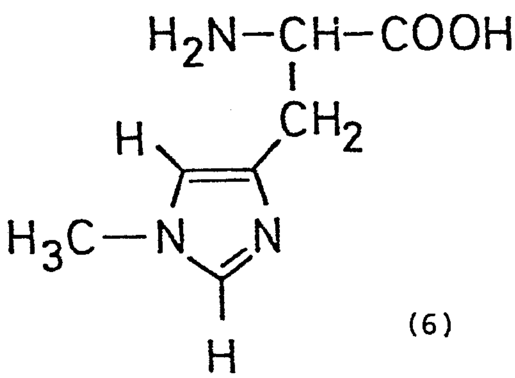

(6)

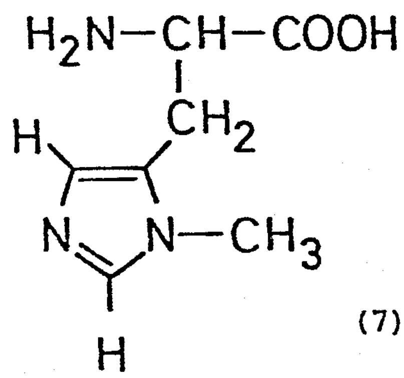

(7)

3-Methylhistidin 1-Methylhistidin

$N^{\tau}$-Methylhistidin $N^{\pi}$-Methylhistidin

$N^{\pi}$-Methylhistidin wurde schon 1938 als Bestandteil des Anserins erkannt, einem Dipeptid der Struktur ß-Alanyl-L-$N^{\pi}$-methylhistidin, das in Fleischextrakten nachgewiesen wurde.

Die freie Aminosäure $N^{\pi}$-Methylhistidin wurde - wie auch die $N^{\tau}$-Methyl-Verbindung - erstmals aus Urin isoliert. Aus Walfischfleisch konnte man die zum Anserin isomere Verbindung, das β-Ala-L-$N^{\tau}$-Methyl-His isolieren.

Die Aminosäure $N^{\tau}$-Methylhistidin ist Bestandteil der myofibrillären Proteine Actin und Myosin, die praktisch das gesamte, im Körper gebildete 3-Methylhistidin beinhalten. Die Methylierung erfolgt erst nach dem Einbau des Histidins in die jeweilige Peptidkette.

Über Versuche zur Herstellung von $N^{\tau}$- und $N^{\pi}$-alkylierten Histidinderivaten wurde in der Literatur verschiedentlich berichtet. Man hat versucht, im Verlauf einer Totalsynthese des Moleküls den entsprechend substituierten Stickstoff einzuführen. Dies wurde für die $N^{\tau}$-Histidine wiederholt versucht (H.H. Tallan, W.H. Stein and S. Moore, J. Biol. Chem. 206, 825 (1954); H. Rinderknecht, T. Rebane and V. Ma, J. Org. Chem. 293, 1968 (1964); P.K. Martin, H.R. Matthews, H. Rapoport and G. Thyagarajan, J. Org. Chem. 33, 3758 (1968)). Die Versuche scheiterten jedoch am nicht-stereospezifischen Verlauf der Reaktionen bzw. am Fehlen einer brauchbaren Trennmethode für die anfallenden racemischen Gemische.

Man hat weiterhin versucht, durch direkte Alkylierung des Histidins und geeigneter Derivate die beiden isomeren Alkylverbindungen zu erhalten. Mit den üblichen Alkylierungsverfahren wurden in niedrigen Ausbeuten Gemische der beiden Isomeren erhalten, die nicht trennbar sind.

Der vorliegenden Erfindung liegt somit die Aufgabe zugrunde, einen praktikablen Syntheseweg zur Darstellung von $N^{\tau}$-substituierten Histidinderivaten, insbesondere von optisch reinen $N^{\tau}$-substituierten Histidinderivaten, zur Verfügung zu stellen. Weiterhin sollen neue $N^{\tau}$-substituierte Histidinderivate zur Verfügung gestellt werden und ihre Verwendung zur Herstellung von Protein- und Enzymderivaten, wie von Polypeptid- und Peptidanalogen, und insbesondere zur Herstellung hochaktiver Hormonderivate. Dabei soll nach Methoden der klassichen Peptidsynthese gearbeitet und aufwendige Reinigungsschritte im Verlauf der Synthese nach Möglichkeit vermieden werden. Die Synthese soll hinsichtlich Ausbeute, Synthesedauer und Schutzgruppeneinsatz optimiert werden.

Gegenstand der Erfindung ist ein Verfahren zur Herstellung von $N^{\tau}$-substituierten Histidinderivaten der allgemeinen Formel 1

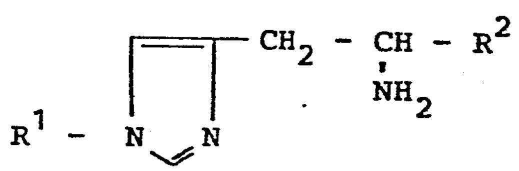

(1)

worin
$R^1$ eine $C_1$-$C_6$-Alkylgruppe, eine $C_3$-$C_6$-Cycloalkylgruppe, eine $C_1$-$C_6$-$\omega$-Halogenalkylgruppe, eine $C_1$-$C_6$-$\omega$-Aminoalkylgruppe, die alle durch eine Cyano-, Nitro-, $C_1$-$C_3$-Alkoxy-, $-COOC_nH_{2n+1}-$ (worin n für 1 bis 3 steht) Gruppe substituiert sein können, eine $C_1$-$C_6$-Alkenyl-, eine $C_3$-$C_6$-Cycloalkenyl- oder eine $C_1$-$C_6$-Alkylenarylgruppe bedeutet und

$R^2$ ein Wasserstoffatom, eine $C_1$-$C_6$-Alkylgruppe oder eine $-COOR^3$-Gruppe, worin $R^3$ für ein Wasserstoffatom, eine Aminogruppe oder eine $C_1$-$C_6$-Alkylgruppe steht, bedeutet,

das dadurch gekennzeichnet ist, daß man ein Histidinderivat der allgemeinen Formel 2, worin $R^2$ die bei Formel 1 gegebene Bedeutung besitzt, mit Phosgen unter Bildung des Zwischenprodukts der allgemeinen Formel 3 gemäß folgender Gleichung

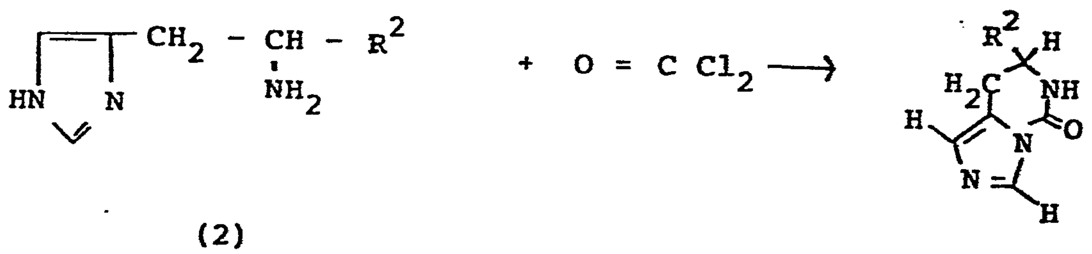

(2) (3)

in Anwesenheit eines organischen Lösungsmittels bei einer Temperatur im Bereich von $-10^{\circ}C$ bis $-50^{\circ}C$ und in Anwesenheit eines Mittels zum Binden der Säure umsetzt, das erhaltene Produkt mit einem Überschuß eines Alkylhalogenids der Formel 4

$$R^1 - Hal \qquad (4)$$

worin $R^1$ die obige Bedeutung besitzt und Hal für ein Halogenatom steht,

unter Bildung eines (7S)-5,6,7,8-Tetrahydro-7-alkoxycarbonyl-2-$R^1$-subst.-5-oxoimidazo-pyrimidiniumhalogenids der folgenden Formel 5

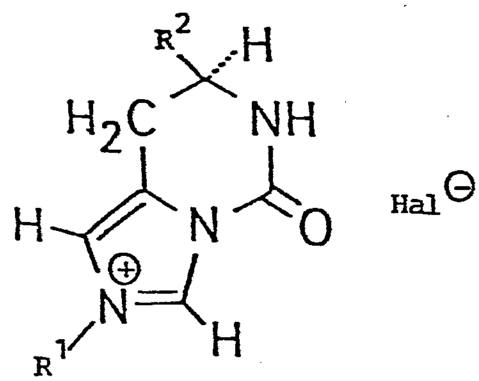

(5)

worin $R^1$ und $R^2$ die oben gegebenen Bedeutungen besitzen und Hal ein Halogenatom bedeutet, alkyliert,

das $N^{\tau}$-substituierte Histidin der allgemeinen Formel 1 durch Behandlung mit Säure hydrolysiert und aus dem Reaktionsgemisch isoliert.

Gegenstand der Erfindung sind weiterhin $N^{\tau}$-substituierte Histidinderivate der allgemeinen Formel 1

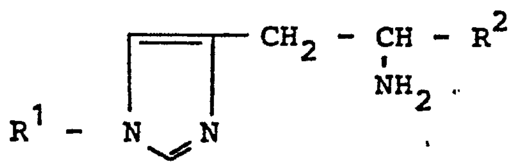

(1)

worin

$R^1$ eine $C_1$-$C_6$-Alkylgruppe, eine $C_3$-$C_6$-Cycloalkylgruppe, eine $C_1$-$C_6$-$\omega$-Halogenalkylgruppe, eine $C_1$-$C_6$-$\omega$-Aminoalkylgruppe, die alle durch eine Cyano-, Nitro-, $C_1$-$C_3$-Alkoxy-, $-COOC_nH_{2n+1}$- (worin n für 1 bis 3 steht) Gruppe substituiert sein können, eine $C_1$-$C_6$-Alkenyl-, eine $C_3$-$C_6$-Cycloalkenyl- oder eine $C_1$-$C_6$-Alkylenarylgruppe bedeutet und

$R^2$ ein Wasserstoffatom, eine $C_1$-$C_6$-Alkylgruppe oder eine $-COOR^3$-Gruppe, worin $R^3$ für ein Wasserstoffatom, eine Aminogruppe oder eine $C_1$-$C_6$-Alkylgruppe steht, bedeutet,

mit Ausnahme der Verbindungen:

worin $R^1$ eine Methyl- oder Ethylgruppe bedeutet und

$R^2$ eine Carboxylgruppe bedeutet.

Schließlich betrifft die Erfindung außerdem die Verwendung der $N^{\tau}$-substituierten Histidinderivate gemäß der Erfindung zur Herstellung von histidinanalogen Proteinderivaten und

und Enzymderivaten, insbesondere zur Herstellung von Peptid- oder Polypeptidderivaten, besonders bevorzugt zur Herstellung von histidinanalogen Gonadoliberinderivaten.

In den obigen Formeln (1), (4) und (5) kann $R^1$ eine $C_1$-$C_6$-Alkylgruppe sein. Die Alkylgruppe kann geradlinig oder verzweigt sein, und Beispiele für Alkylgruppen sind die Methyl-, Ethyl-, n-Propyl-, Isopropyl-, n-Butyl-, Isobutyl- und tert.-Butylgruppen. Von diesen Gruppen sind die Methylgruppe und die tertiäre und sekundäre Butylgruppe besonders bevorzugt. $R^1$ kann weiterhin eine $C_3$-$C_6$-Cycloalkylgruppe, wie eine Cyclopropyl- oder Cyclopentylgruppe, eine $C_1$-$C_3$-$\omega$-Halogenalkylgruppe oder $C_1$-$C_6$-$\omega$-Aminoalkylgruppe bedeuten. Bevorzugte $\omega$-substituierte Alkylgruppen sind die oben erwähnten Alkylgruppen, die in $\omega$-Stellung mit einem Halogen, wie beispielsweise Chlor, Fluor, Brom oder Jod, substituiert sind oder die in $\omega$-Stellung mit einer Aminoalkylgruppe substituiert sind. Diese genannten Gruppen können unsubstituiert oder weiter durch die oben aufgeführten Substituenten substituiert sein. Von den oben für $R^1$ angegebenen Bedeutungen ist $R^1$ bevorzugt eine geradlinige oder verzweigte $C_1$-$C_6$-Alkylgruppe oder eine geradlinige oder verzweigte $C_1$-$C_6$-Alkylenarylgruppe. Besonders bevorzugt ist $R^1$ eine Methyl-, Ethyl- oder Benzylgruppe. In der $C_1$-$C_6$-Alkylenarylgruppe kann die Arylgruppe eine Phenylgruppe oder eine Naphthylgruppe sein. Die aromatischen Gruppen können weiterhin durch zwei oder drei Substituenten, wie eine $C_1$-$C_3$-Alkylgruppe, ein Halogenatom (Fluor, Brom, Chlor oder Jod), eine Trifluormethylgruppe, eine Nitrogruppe, eine Cyanogruppe oder eine $-COO-C_1-C_3$-Alkylgruppe, substituiert sein.

In den obigen Formeln bedeutet $R^2$ ein Wasserstoffatom, eine geradlinige oder verzweigte $C_1$-$C_6$-Alkylgruppe, beispielsweise eine Methyl-, Ethyl-, n-Propyl-, Isopropyl-, n-Butyl-, Isobutyl- oder tert.-Butylgruppe. $R^2$ kann weiterhin eine $-COOR^3$-Gruppe sein, worin $R^3$ für ein Wasserstoffatom, eine

Aminogruppe oder eine $C_1$-$C_6$-Alkylgruppe steht. Wenn $R^3$ eine $C_1$-$C_6$-Alkylgruppe bedertet, kann diese geradlinig oder verzweigt sein. Bevorzugt ıst $R^2$ ein Wasserstoffatom, eine $C_1$-$C_3$-Alkylgruppe und besonders bevorzugt ein Wasserstoffatom.

Überraschenderweise wurde gefunden, daß ein sehr stabiler, vollreversibler Schutz der $N^{\tau}$-Funktion des Imidazolrestes am Histidin möglich ist, indem man Histidinmethylester in Gegenwart einer Base bei tiefen Temperaturen mit Phosgen umsetzt. In der folgenden Gleichung ist der Reaktionsverlauf anhand des Methylesters des Histidins dargestellt.

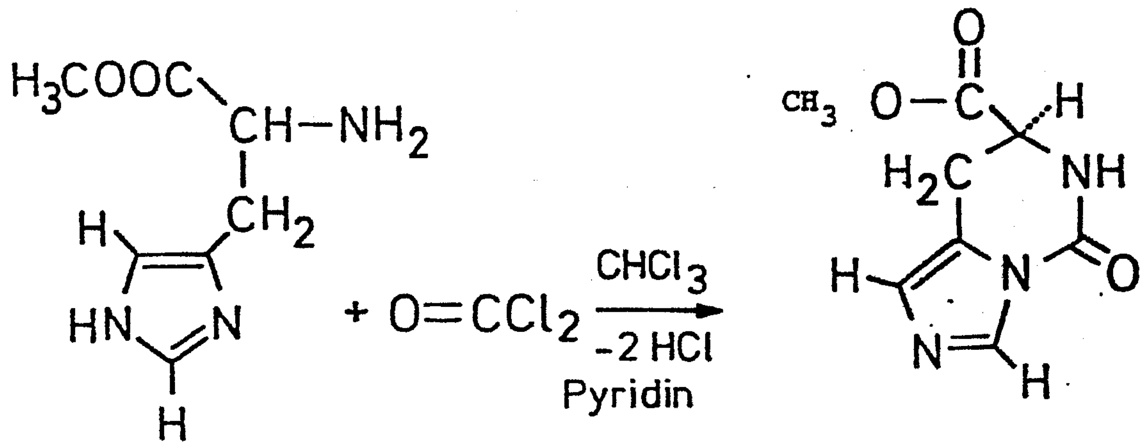


Darstellung von (7 S)-5,6,7,8-Tetrahydro-7-methoxycarbonyl-5-oxoimidazo(1.5-c)-pyrimidin

Bei der ersten Stufe des Verfahrens verwendet man als Ausgangsmaterial die oben aufgeführten Verbindungen der Formel 2. Die Umsetzung der Verbindung der Formel 2 mit Phosgen erfolgt in einem organischen Lösungsmittel, das gegenüber Phosgen inert ist und bei der Reaktionstemperatur noch flüssig ist bzw. zusammen mit den anderen Reaktionsteilnehmern ein flüssiges Gemisch bildet. Als Lösungsmittel kann man beispielsweise Kohlenwasserstoffe, halogenierte Kohlenwasserstoffe, wie Methylenchlorid, Chloroform, Tetrachlorethan, Trichlorethan, Ether, wie Dioxan, Diethylether, Tetrahydrofuran, oder dipolare aprotische Lösungsmittel, wie Dimethylformamid, Dimethylsulfoxid oder Hexamethylphosphorsäuretriamid, verwenden. Bevorzugt werden Kohlenwasserstoffe und Ether verwendet. Die Reaktion wird bei Temperaturen im Bereich von -10°C bis -50°C, vorzugsweise -20°C bis -40°C, besonders bevorzugt bei etwa -35°C, durchgeführt.

Bei der Umsetzung verwendet man ein Mittel zum Binden der Salzsäure. Als Mittel zum Binden der Salzsäure kann man übliche Basen verwenden. Bevorzugt wird Pyridin, Triethylamin oder Trimethylamin eingesetzt.

Im allgemeinen leitet man das Phosgen bei der gewünschten Temperatur, beispielsweise durch Kühlen in einem Methanol/Trockeneisbad, in die Lösung ein und rührt dann noch während einer Zeit von 10 bis 60 Minuten unter Kühlen. Das erhaltene Reaktionsgemisch wird dann in gekühltes Nichtlösungsmittel für das Reaktionsprodukt gegossen, beispielsweise Ether, Hexan etc.

Im allgemeinen fällt dann ein feiner weißer, leicht zerschließender Niederschlag an dem Reaktionsprodukt der Formel 3 aus. Der Niederschlag wird abgesaugt und mit den Lösungsmitteln, mit denen er ausgefällt wurde, gewaschen. Beispielsweise läßt sich die Verbindung 3, worin $R^2$ eine Methylgruppe bedeutet, aus Methanol in schönen Kristallen und hoher Reinheit kristallisieren. Sie ist bei Raumtemperatur und Lichteinfluß stabil, und im Verlauf von zwei Jahren wird keine Zersetzung beobachtet.

Die Alkylierung am $N^{\tau}$-Atom des Imidazolrestes von 5,6,7,8-Tetrahydro-7-alkoxycarbonyl-5-oxoimidazol-1,5,10-pyrimidin der Formel 3 gelingt leicht und in hohen Ausbeuten, wenn man die Substanz mit einem Überschuß an einem Alkylhalogenid der Formel 4 umsetzt.

Bevorzugt verwendet man einen zehnfachen Überschuß an frisch destilliertem Alkylhalogenid, wie beispielsweise dem

Jodid oder dem Chlorid oder dem Bromid. Als Alkylierungsmittel kann man somit beispielsweise die Jodide oder Bromide verwenden. Bevorzugt verwendet man die Jodide. Beispiele für Alkylierungsmittel sind Methyl-, Ethyl-, n-Propyl-, Isopropyl- und n-Butyljodid.

Im allgemeinen wird die Substanz der Formel 3 in dem Überschuß an Alkylhalogenid am Rückfluß erhitzt. Die entstehenden Alkylpyrimidiniumhalogenide kristallisieren im allgemeinen nach Zugabe eines organischen unpolaren Lösungsmittels, beispielsweise Methylenchlorid, Chloroform, Halogenkohlenwasserstoffe.

Die Freisetzung der in $N^{\tau}$-Position alkylierten Histidinderivate gelingt leicht und in fast quantitativer Ausbeute durch Kochen mit einer Mineralsäure. Als Mineralsäure verwendet man im allgemeinen starke Säuren, wie Salzsäure, Schwefelsäure oder Perchlorsäure. Die Konzentration der Säure beträgt im allgemeinen 4N bis 8N, bevorzugt 6N. Man kann jedoch auch Konzentrationen, die unterhalb des angegebenen Bereichs liegen, verwenden.

Die $N^{\tau}$-Alkylhistidinderivate kristallisieren als Dihydrochlorid aus Ethanol nach Zugabe von Ether.

Es sind bei Raumtemperatur stabile und nicht-lichtempfindliche Verbindungen. Setzt man dagegen die freie Base frei und hält sie in Lösung bei Raumtemperatur, so tritt eine Zersetzung unter Braunfärbung der Lösung ein, wie man sie auch bei freiem Histidinmethylester beobachten kann.

Verwendet man bei dem erfindungsgemäßen Verfahren als Ausgangsmaterial ein Racemat, so erhält man als Endprodukt auch ein racemisches Gemisch. Dieses racemische Gemisch läßt sich nach an sich bekannten Verfahren trennen. Verwendet man bei dem erfindungsgemäßen Verfahren die reinen L-oder reinen D-Verbindungen, so erhält man auch die reinen L- bzw. reinen D-Verbindungen. Das erfindungsgemäße Verfahren ermöglicht daher auf einfache Art und Weise die Synthese von optisch reinen Verbindungen.

Es wurde weiterhin gefunden, daß die neu synthetisierten $N^{\tau}$-substituierten Histidinderivate der allgemeinen Formel 1 zur Herstellung von biologisch wichtigen Substanzen, sogenannter Biomoleküle, verwendet werden können.

Die erfindungsgemäßen Verbindungen können zur Synthese von histidinanalogen Proteinderivaten und Enzymderivaten eingesetzt werden. Bevorzugt verwendet man sie zur Herstellung von Peptid- oder Polypeptidderivaten, besonders bevorzugt zur Herstellung von histidinanalogen Gonadoliberinderivaten. Es hat sich gezeigt, daß beispielsweise die TRH-freisetzende Aktivität von Tripeptiden, die anstelle von Histidin das $N^{\tau}$-Methylhistidinderivat enthalten, um ein Vielfaches höher ist als die der Histidin enthaltenden Tripeptide, wie aus der folgenden Tabelle hervorgeht.

Tabelle

| Verbindung | Aktivität, bezogen auf TRH = 100% |
|---|---|
| L-Pyr-1-Me-L-His-L-Pro-$NH_2$ | 0,1 |
| L-Pyr-1-Me-L-His-L-Pro-$NH_2$ | 0,04 |
| L-Pyr-1-Me-L-His-L-Pro-$NH_2$ | 0,04 |
| L-Pyr-3-Me-L-His-L-Pro-$NH_2$ | 800 |
| L-Pyr-3-Me-L-His-L-Pro-$NH_2$ | 800 |
| L-Pyr-3-Me-L-His-L-Pro-$NH_2$ | 800 |

Die erfindungsgemäßen Verbindungen können somit nach an sich bekannten Verfahren zur Synthese von allen Biomolekülen verwendet werden, beispielsweise für die Synthese hypothalamischer Hormone (vgl. Derek Gupta und Wolfgang Voelter, Proceedings of the European Colloquium on Hypothalamic Hormones held at Tübingen in February 1974, Verlag Chemie). Alle in der genannten Literaturstelle aufgeführten Verfahren können auch mit den erfindungsgemäßen Verbindungen durchgeführt werden.

Im folgenden wird die Verwendung von $N^{\tau}$-alkylierten Histidinderivaten zur Darstellung histidinanaloger Gonadoliberinderivate beschrieben.

Als Modellsubstanz für eine Peptidsynthese unter Verwendung der erfindungsgemäß dargestellten Alkylhistidine wurde Gonadotropin-"Releasing"-Hormon (Gn-RH,LH/FSH-RH) gewählt. Seit seiner Isolierung und Strukturaufklärung ist dieses Dekapeptid, das im Hypothalamus gebildet wird und in der Hypophyse die Ausschüttung der gonadotropen Hormone bewirkt, Gegenstand intensiver chemischer und medizinischer Forschung.

In der Beschreibung und in den folgenden Beispielen werden die folgenden Abkürzungen verwendet:

| | | |
|---|---|---|
| Adpoc | = | 1-(1-Adamantyl)-1-methylethoxycarbonyl |
| Boc | = | tert.-Butyloxycarbonyl |
| Bzl | = | Benzylether |
| DCC | = | N,N'-Dicyclohexylcarbodiimid |
| DMF | = | Dimethylformamid |
| EE | = | Essigsäure ethylester |
| EtOH | = | Ethanol |
| Et | = | Ethyl |
| FD-MS | = | Felddesorptions-Massenspektrometrie |
| HOBt | = | 1-Hydroxybenzotriazol |
| HAc | = | Essigsäure |
| Mbs | = | p-Methoxybenzolsulfonyl |
| Me | = | Methyl |
| MeOH | = | Methanol |
| OMe | = | Methylester |
| OBzl | = | Benzylester |
| PFT | = | Puls-Fourier-Transformation |
| OSu | = | N-Hydroxysuccinimidester |
| THF | = | Tetrahydrofuran |
| Z | = | Benzyloxycarbonyl |

Die folgenden Beispiele erläutern die Erfindung.

## Beispiel 1

### 1. L-Histidin-methylester · 2 Hydrochlorid (H-L-His-OMe · 2HCl)

Die Darstellung der Verbindung erfolgt nach N.C. Davis, J. Biol. Chem. 223, 935 (1956).

(Ansatz: 60 g (0,31 Mol L-Histidinhydrochlorid). In Abweichung der Vorschrift wird so lange unter Rückfluß erhitzt, bis sich eine klare Lösung gebildet hat. Dann

### 2. L-Histidin-methylester (H-L-His-OMe)

12,1 g (0,05 Mol) kristallines L-Histidinmethylesterdihydrochlorid wird im Mörser fein zerrieben und in 150 ml absolutem Chloroform suspendiert. Dann leitet man unter Eiskühlung 10 Minuten lang trockenes Ammoniakgas ein und filtriert das ausgefallene Ammoniumchlorid ab. Die klare Chloroformphase wird im Vakuum zur Trockene eingeengt. Der als farbloses Öl anfallende freie Methylester wird kühl und dunkel gelagert.

Ausbeute: 8,45 g (quantitativ)
Die Verbindung wird ohne weitere Charakterisierung umgesetzt.

### 3. (+)-(S)-5,6,7,8-Tetrahydro-7-(methoxycarbonyl)-5-oxo-imidazo-[1.5-c]pyrimidin

3,38 g (20 mMol) L-Histidinmethylester und 3,16 g (40 mMol) Pyridin (destilliert über Ninhydrin) werden in 100 ml Chloroform gelöst und mit Methanol/Trockeneis auf -30 bis -40°C gekühlt. Bei dieser Temperatur leitet man für 15 Minuten einen schwachen Strom HCl-freies Phosgen ein

und rührt noch 15 Minuten in der Kälter. Dann gießt man den Ansatz in 400 ml kalten Ether/Hexan (2:1). Es fällt ein feiner, weißer, leicht zerfließender Niederschlag aus, der abgesaugt und mit Ether gewaschen wird. Der Niederschlag wird in 200 ml 1 N Natriumbicarbonatlösung aufgenommen und 6mal mit je 50 ml Chloroform extrahiert. Die $CHCl_3$-Phase wird über Natriumsulfat getrocknet und im Vakuum eingeengt. Verbleibende Spuren von Pyridin werden im Hochvakuum entfernt. Die Substanz kristallisiert aus Methanol in schönen, farblosen Kristallen.

Ausbeute: 3,39 g (87% d.Th.)
Fp.: 160 - 162°C

4. (+)-(S)-2-Methyl-5,6,7,8-tetrahydro-7-(methoxycarbonyl)-5-oxoimidazo[1.5-c]pyrimidin·Hydrojodid

3 g (15,3 mMol) des 5,6,7,8-Tetrahydro-7-(methoxycarbonyl)-5-oxoimidazopyrimidins werden in 30 ml absolutem Dimethylformamid gelöst und mit 9,34 ml (150 mMol) frisch destilliertem Methyljodid versetzt. Man kocht 2,5 Stunden unter Rückfluß, läßt auf Zimmertemperatur abkühlen und gießt in 150 ml Ether. Die kristalline Verbindung wird abgesaugt, mit wenig kaltem Methanol und Ether gewaschen und aus Methanol umkristallisiert. Die Substanz sollte unter Lichtschutz und in der Kälte aufbewahrt werden.

Ausbeute: 4,7 g (91% d.Th.)
Fp.: 171 - 172°C

5. L-$N^{\tau}$-Methylhistidin·2 Hydrochlorid (H-L-His($N^{\tau}$-Me)OH·2 HCl)

3,37 g (10 mMol) (+)-(7S)-5,6,7,8-Tetrahydro-7-(methoxycarbonyl)-2-methyl-5-oxoimidazo[1.5-c]pyrimidin·Hydro-

jodid wird in 40 ml 6 N HCl so lange unter Rückfluß erhitzt, bis durch Dünnschichtchromatographie keine Ausgangsverbindung mehr nachweisbar ist (6 bis 8 Stunden). Die Salzsäure wird dann im Vakuum abgezogen, der ölige Rückstand in Ethanol aufgenommen und durch Zugabe von Ether kristallisiert.

Ausbeute: 2,0 g (83% d.Th.)
Fp.: 258°C

## Beispiel 2

1. (+)-(7S)-2-Ethyl-5,6,7,8-tetrahydro-7-(methoxycarbonyl)-5-oxoimidazo[1.5-c]pyrimidin·Hydrojodid

3,9 g (20 mMol) des (7S)-5,6,7,8-Tetrahydro-7-(methoxycarbonyl)-5-oxoimidazopyrimidins werden mit 31,1 g (200 mMol) frisch destilliertem Ethyljodid, erhalten gemäß Beispiel 1, und 40 ml absolutem Dimethylformamid 3 Stunden unter Rückfluß erhitzt. Dann läßt man auf Raumtemperatur abkühlen und gibt Ether zu, bis eine Trübung eintritt. Das Produkt fällt bei 4°C in Form gelber Kristalle an und wird aus Methanol umkristallisiert. Die Verbindung sollte dunkel und kühl gelagert werden, da bei Licht Dunkelfärbung und Zersetzung eintritt.

Ausbeute: 6,24 g (89% d.Th.)
Fp.: 140°C (Zersetzung)

2. L-$N^{\tau}$-Ethyl-histidin·2 Hydrochlorid (H-L-His($N^{\tau}$-Et)-OH·2HCl

1,75 g (5 mMol) des nach obiger Vorschrift dargestellten (+)-(7S)-2-Ethyl-5,6,7,8-tetrahydro-7-(methoxycarbonyl)-5-oxoimidazopyrimidin·Hydrojodids werden in 20 ml 6 N Salzsäure 6 Stunden unter Rückfluß erhitzt. Dann wird die Salzsäure im Hochvakuum vollständig entfernt, der

rot gefärbte, ölige Rückstand in Ethanol aufgenommen, getrocknet und durch Zugabe von Ether kristallisiert.

Ausbeute: 0,97 g (76% d.Th.)
Fp.: 235°C

B e i s p i e l 3

1. (-)-(R)-5,6,7,8-Tetrahydro-7-methoxycarbonyl-5-oxo-imidazo-(1.5-c)-pyrimidin

Man löst 1,7 g (10 mMol) D-Histidinmethylester und 1,58 g (20 mMol, 1,61 ml) destilliertes Pyridin in 50 ml absolutem Chloroform, kühlt mit Methanol/Trockeneis auf -40 bis -50°C und leitet bei dieser Temperatur für 15 Minuten einen schwachen Strom HCl-freies Phosgen ein. Man rührt noch 15 Minuten in der Kälte und gießt in 200 ml kalten Ether/Hexan (2:1) ein. Es fällt ein weißer, zerfließlicher Niederschlag, der rasch abgesaugt und mit Ether gewaschen wird. Der Niederschlag wird in 100 ml 1 N $Na_2CO_3$-Lösung aufgenommen und 6mal mit Chloroform extrahiert. Nach dem Trocknen der organischen Phase über Natriumsulfat wird das Lösungsmittel abgezogen, eventuell vorhandene Spuren von Pyridin werden im Hochvakuum entfernt. Die Substanz kristallisiert aus Methanol.

Ausbeute: 1,45 g (85% d.Th.)
Fp.: 164°C

B e i s p i e l 4

Peptidsynthese unter Verwendung der $N^{\tau}$-Alkylhistidine

1. N-Benzyloxycarbonyl-L-pyroglutamyl-$N^{\tau}$-methyl-L-histidin (Z-L-Pyr-L-His($N^{\tau}$-Me)-OH)

1,48 g (4,11 mMol) Z-L-Pyr-OSu werden in 5 ml Dioxan gelöst und mit einer Lösung von 0,69 g (4,11 mMol) H-L-His-($N^{\tau}$-Me)-OH und 0,43 g (4,1 mMol) wasserfreiem Natriumcarbonat in 5 ml Wasser versetzt. Man rührt 4 Stunden bei Raumtemperatur und neutralisiert dann mit 0,1 N Salzsäure. Das Lösungsmittel wird im Hochvakuum abgezogen, der Rückstand in wenig Methanol aufgenommen und das nicht gelöste Kochsalz abfiltriert. Da die Kristallisation aus 30% Methanol (wie bei der nichtalkylierten Verbindung) nicht gelingt, wird das Produkt durch zweimaliges Umfällen aus Methanol/Ether gewonnen.

Ausbeute: 1,05 g (62%)
Fp.: 144°C

2. N-Benzyloxycarbonyl-L-pyroglutamyl-$N^{\tau}$-ethyl-L-histidin (Z-L-Pyr-L-His($N^{\tau}$Et)-OH)

0,72 g (2 mMol) Z-L-Pyr-OSu werden in 4 ml Dioxan gelöst. Man vereinigt mit einer Lösung von 0,36 g (2 mMol) H-L-His($N^{\tau}$-Et)-OH und 0,21 g (2 mMol) wasserfreiem Natriumcarbonat in 3 ml Wasser und rührt 6 Stunden bei Raumtemperatur. Nach Beendigung der Reaktion neutralisiert man mit 0,1 N Salzsäure und engt den Ansatz im Hochvakuum zur Trockene ein. Das zurückbleibende Öl wird in wenig Methanol aufgenommen, das nicht gelöste Kochsalz abfiltriert und das Produkt durch Umfällen aus Methanol/Ether gewonnen.

Ausbeute: 490 mg (58%)
Fp.: 156 - 158°C

3. N-Benzyloxycarbonyl-L-pyroglutamyl-$N^{\tau}$-methyl-L-histidyl-L-prolinamid (Z-L-Pyr-L-His($N^{\tau}$-Me)-L-Pro-$NH_2$)

414 mg (1 mMol) Z-L-Pyr-L-His($N^{\tau}$-Me)-OH, 220 mg (1,1 mMol) Dicyclohexylcarbodiimid und 135 mg (1 mMol) 1-Hydroxybenzotriazol werden in 10 ml DMF gelöst und bei 0°C mit einer Lösung von 150 mg (1 mMol) L-Pro-$NH_2$·HCl und 100 mg (1 mMol) N-Methylmorpholin in wenig DMF versetzt. Man rührt 1 Stunde bei 0°C und über Nacht bei Raumtemperatur. Danach wird in der Kälte vom ausgefallenen Dicyclohexylharnstoff abfiltriert, das Lösungsmittel im Hochvakuum abgezogen und der Rückstand in Essigester aufgenommen. Man wäscht mit Bicarbonat und Wasser, trocknet über Natriumsulfat und fällt das Produkt aus Essigester/Methanol (9:1) durch Zugabe von Ether aus.

Ausbeute: 326 mg (64%)

4. L-Pyroglutamyl-$N^{\tau}$-methyl-L-histidyl-L-prolinamid (H-L-Pyr-L-His($N^{\tau}$-Me)-L-Pro-$NH_2$)

255 mg (0,5 mMol) geschütztes Tripeptid werden in 15 ml Methanol und 5 ml Wasser gelöst und mit 50 mg Pd/Aktivkohle (10% Pd) versetzt. Es wird 2 Stunden bei pH 4,5-5

(Essigsäure) im Durchflußverfahren hydriert. Die Aktivkohle wird über Celite abfiltriert, das Lösungsmittel abgezogen und der Rückstand in Wasser aufgenommen. Man versetzt die Lösung mit 1,5 g Ionenaustauscher (OH-Form), läßt 20 Minuten bei Raumtemperatur stehen und filtriert ab.

Die wäßrige Lösung wird gefriergetrocknet.

Ausbeute: 154 mg (82%)
Die Reinigung erfolgt durch Ionenaustauschchromatographie an Sephadex C-50.

5. N-Benzyloxycarbonyl-L-pyroglutamyl-$N^{\tau}$-ethyl-L-histidyl-prolinamid (Z-L-Pyr-L-His-($N^{\tau}$-Et)-L-Pro-$NH_2$)

215 mg (0,5 mMol) Z-L-Pyr-L-His($N^{\tau}$-Et)-OH, 110 mg (0,55 mMol) Dicyclohexylcarbodiimid und 70 mg (0,5 mMol) 1-Hydroxybenzotriazol werden in 8 ml Dimethylformamid gelöst und auf $0^{o}C$ gekühlt. Dann wird mit einer Lösung von 75 mg (0,5 mMol) L-Pro-$NH_2$·HCl und 50 mg (0,5 mMol) N-Methylmorpholin in wenig DMF versetzt, 1 Stunde in der Kälte und 12 Stunden bei Raumtemperatur gerührt. Nach dem Abfiltrieren des Dicyclohexylharnstoffs wird das Lösungsmittel im Hochvakuum entfernt und der Rückstand in Essigester (durch Zugabe einiger Tropfen Methanol) gelöst. Man wäscht mit Natriumbicarbonat und Wasser, trocknet und fällt das Produkt aus Essigester/Methanol (9:1) durch Zugabe von Ether aus.

Ausbeute: 139 mg (53%)

6. L-Pyroglutamyl-$N^{\tau}$-ethyl-L-histidyl-L-prolinamid (L-Pyr-L-His($N^{\tau}$-Et)-L-Pro-$NH_2$)

104 mg (0,2 mMol) des benzyloxycarbonylgeschützten Tripeptids werden in 10 ml Methanol und 5 ml Wasser gelöst und mit 50 mg PD/Aktivkohle (10%) versetzt. Man hydriert 2 bis 3 Stunden im Durchflußverfahren (pH 4,5 - 5, Essigsäure), filtriert den Katalysator über Celite ab und entfernt das Lösungsmittel im Vakuum. Der Rückstand wird in Wasser aufgenommen, 20 Minuten mit Ionenaustauscher behandelt (OH-Form) und dann lyophilisiert.

Ausbeute: 60 mg (76%)

Verfahren zur Herstellung von $N^{\tau}$-substituierten Histidinderivaten, $N^{\tau}$-substituierte Histidinderivate und ihre Verwendung

## PATENTANSPRÜCHE

1. Verfahren zur Herstellung von $N^{\tau}$-substituierten Histidinderivaten der allgemeinen Formel 1

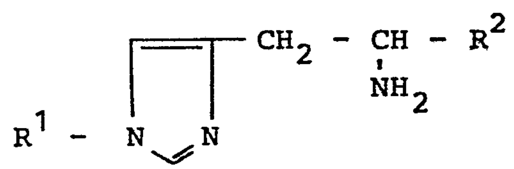

(1)

worin

$R^1$ eine $C_1$-$C_6$-Alkylgruppe, eine $C_3$-$C_6$-Cycloalkylgruppe, eine $C_1$-$C_6$-$\omega$-Halogenalkylgruppe, eine $C_1$-$C_6$-$\omega$-Aminoalkylgruppe, die alle durch eine Cyano-, Nitro-, $C_1$-$C_3$-Alkoxy-, $-COOC_nH_{2n+1}$- (worin n für 1 bis 3 steht) Gruppe substituiert sein können, eine $C_1$-$C_6$-Alkenyl-, eine $C_3$-$C_6$-Cycloalkenyl- oder eine $C_1$-$C_6$-Alkylenarylgruppe bedeutet und

$R^2$ ein Wasserstoffatom, eine $C_1$-$C_6$-Alkylgruppe oder eine $-COOR^3$-Gruppe, worin $R^3$ für ein Wasserstoffatom, eine Aminogruppe oder eine $C_1$-$C_6$-Alkylgruppe steht, bedeutet,

dadurch gekennzeichnet, daß man ein Histidinderivat der allgemeinen Formel 2, worin $R^2$ die bei Formel 1 gegebene Bedeutung besitzt, mit Phosgen unter Bildung des Zwischenprodukts der allgemeinen Formel 3 gemäß folgender Gleichung

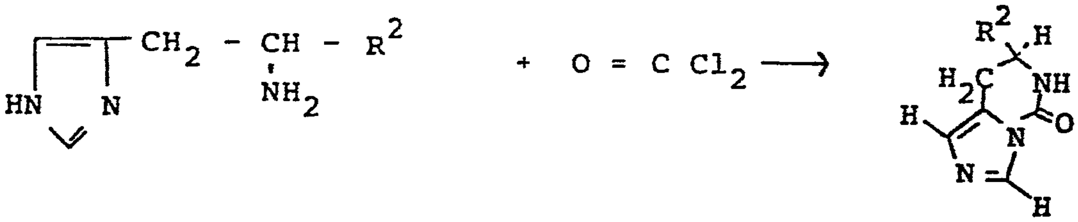

(2) (3)

in Anwesenheit eines organischen Lösungsmittels bei einer Temperatur im Bereich von $-10^oC$ bis $-50^oC$ und in Anwesenheit eines Mittels zum Binden der Säure umsetzt, das erhaltene Produkt mit einem Überschuß eines Alkylhalogenids der Formel 4

$$R^1 - Hal \qquad (4)$$

worin $R^1$ die obige Bedeutung besitzt und Hal für ein Halogenatom steht,

unter Bildung eines (7S)-5,6,7,8-Tetrahydro-7-alkoxycarbonyl-2-$R^1$-subst.-5-oxoimidazo-pyrimidiniumhalogenids der folgenden Formel 5

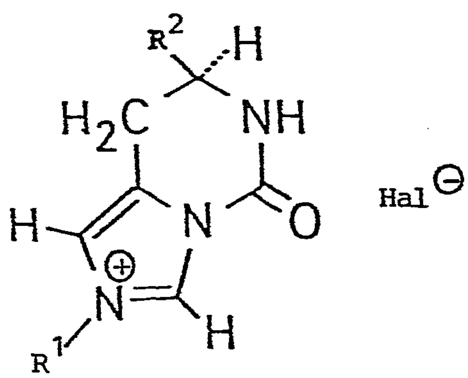

(5)

worin $R^1$ und $R^2$ die oben gegebenen Bedeutungen besitzen und Hal ein Halogenatom bedeutet, alkyliert,

das $N^{\tau}$-substituierte Histidin der allgemeinen Formel 1 durch Behandlung mit Säure hydrolysiert und aus dem Reaktionsgemisch isoliert.

2. Verfahren nach Anspruch 1, dadurch gekennzeichnet, daß man als Mittel zum Binden der Säure eine in dem Reaktionsmedium lösliche anorganische oder organische Base verwendet.

3. Verfahren nach Anspruch 1 oder 2, dadurch gekennzeichnet, daß man zur Herstellung eines L-Histidinderivats der allgemeinen Formel 1 ein L-Histidinderivat der allgemeinen Formel 2 und zur Herstellung eines D-Histidinderivats der allgemeinen Formel 1 ein D-Histidinderivat der allgemeinen Formel 2 als Ausgangsmaterial verwendet.

4. $N^{\tau}$-substituierte Histidinderivate der allgemeinen Formel 1

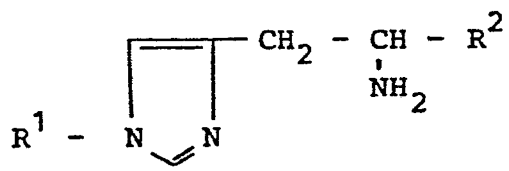

(1)

worin $R^1$ eine $C_1$-$C_6$-Alkylgruppe, eine $C_3$-$C_6$-Cycloalkylgruppe, eine $C_1$-$C_6$-$\omega$-Halogenalkylgruppe, eine $C_1$-$C_6$-$\omega$-Aminoalkylgruppe, die alle durch eine Cyano-, Nitro-, $C_1$-$C_3$-Alkoxy-, $-COOC_nH_{2n+1}$- (worin n für 1 bis 3 steht) Gruppe substituiert sein können, eine $C_1$-$C_6$-Alkenyl-, eine $C_3$-$C_6$-Cycloalkenyl- oder eine $C_1$-$C_6$-Alkylenarylgruppe bedeutet und

$R^2$ ein Wasserstoffatom, eine $C_1$-$C_6$-Alkylgruppe oder eine $-COOR^3$-Gruppe, worin $R^3$ für ein Wasserstoffatom, eine Aminogruppe oder eine $C_1$-$C_6$-Alkylgruppe steht, bedeutet,

mit Ausnahme der Verbindungen:

worin $R^1$ eine Methyl- oder Ethylgruppe bedeutet und

$R^2$ eine Carboxylgruppe bedeutet.

5. Verwendung der $N^{\tau}$-substituierten Histidinderivate der allgemeinen Formel 1 gemäß Anspruch 1 zur Herstellung von histidinanalogen Proteinderivaten und Enzymderivaten.

6. Verwendung nach Anspruch 5, dadurch g e k e n n - z e i c h n e t , daß man Peptid- oder Polypeptidderivate herstellt.

7. Verwendung nach Anspruch 5, dadurch g e k e n n - z e i c h n e t , daß man histidinanaloge Gonadoliberinderivate herstellt.